# EUROPEAN PATENT APPLICATION

(11) **EP 4 335 849 A1**
(43) Date of publication of application: **13.03.2024**
(21) Application number: 22799100.7
(22) Date of filing: 03.05.2022
(51) Int. Cl.: C07D 487/04, A61K 31/519, A61P 35/00, A23L 33/10

(54) **PHARMACEUTICAL COMPOSITION FOR PREVENTION, ALLEVIATION, OR TREATMENT OF CANCER**

(30) Priority: 03.05.2021 KR 20210056988; 03.05.2021 KR 20210056994
(71) Applicant: JBKLAB Co., Ltd., Seongnam-si, Gyeonggi-do 13229 (KR)
(72) Inventor: JANG, Bong Keun, Seongnam-si Gyeonggi-do 13229 (KR)
(74) Representative: Office Freylinger
(86) International application number: PCT/KR2022/006349
(87) International publication number: WO 2022/235057

(57) **Abstract**

The compound according to the present invention exhibits excellent anticancer effects against the triple-negative breast cancer cell line MDA-MB-231 and liver cancer cell lines as well as general cancer cell lines such as A549, SK-OV-3, SK-MEL-2, HCT15, etc., and thus can be advantageously used as an anticancer agent.

## Description

### BACKGROUND OF THE INVENTION

### 1. Field of the Invention

The present invention relates to a pharmaceutical composition for the prevention, alleviation, or treatment of cancer.

### 2. Description of the Related Art

Despite the fact that the incidence of cancer has been increasing with the development of civilization, the treatment of cancer patients still relies on surgical operations, radiation therapy, and chemotherapy by administering anticancer drugs with strong cytotoxicity.

However, these treatments are generally limited to patients with early-stage cancer or specific types of cancer and cause various side effects, so there is a need to develop anticancer drugs that are effective and have fewer side effects while having a safety profile.

In particular, the breast cancer market is known to be a field with unmet medical needs that lack high anticancer effectiveness and safety.

Regarding breast cancer treatment, therapies with various mechanisms targeting patients with different disease characteristics, such as hormone therapy, chemotherapy, and targeted therapy, are being introduced, but there is a steady clinical demand for therapies with excellent anti-cancer efficacy and safety. Among these, triple-negative breast cancer (TNBC), which accounts for 16% of all breast cancers, is difficult to treat due to the poor prognosis after treatment, and there are not enough therapies available to target TNBC (Non-patent reference 1, Rev Peru Med Exp Salud Publica. Oct-Dec 2013;30(4) :649-56) .

Currently, treatments for breast cancer including therapies that inhibit the human epidermal growth factor receptor 2 (hereinafter referred to as 'HER2') gene, which is involved in tumor growth, and anti-hormonal drugs are widely used. However, in the case of TNBC, HER2 receptor, estrogen receptor, and progesterone receptor are all negative, so it does not respond to existing anticancer drugs. Accordingly, a new targeted treatment that can treat safely and efficiently is urgently needed.

Thus, the present inventors have studied using various cancer cell lines and completed the pharmaceutical composition of the present invention, which can be used as a safe and effective anticancer drug.

### SUMMARY OF THE INVENTION

It is an object of the present invention to provide a pharmaceutical composition for preventing or treating cancer comprising a compound represented by formula 1 or 2 described herein as an active ingredient.

It is another object of the present invention to provide an N-phenyl-7H-pyrrolo[2,3-d]pyrimidin-4-amine derivative compound.

It is another object of the present invention to provide a health functional food composition for preventing or treating cancer comprising a compound represented by formula 1 or 2 described herein as an active ingredient.

It is another object of the present invention to provide a method for treating cancer, comprising a step of administering a compound represented by formula 1 or 2 described herein to a subject in need thereof.

It is another object of the present invention to provide a compound represented by formula 1 or 2 described herein for use in the prevention or treatment of cancer.

It is another object of the present invention to provide a use of a compound represented by formula 1 or 2 described herein for the preparation of a medicament for use in the prevention or treatment of cancer.

To achieve the above objects, in an aspect of the present invention, the present invention provides a pharmaceutical composition for preventing or treating cancer comprising a compound represented by formula 1 or 2 below, a stereoisomer thereof, a solvate thereof, a hydrate thereof, or a pharmaceutically acceptable salt thereof as an active ingredient. 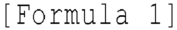 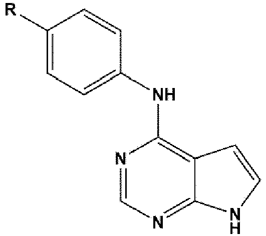 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl; 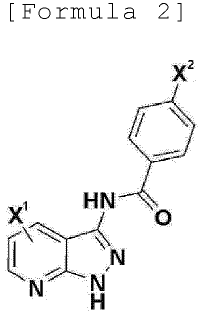
In formula 2 above,
X¹ is halogen, C₁₋₃ alkoxy, C₁₋₃ alkyl, cyano, hydroxy, amino, or piperidine substituted pyrazolyl,
X² is halogen, or methyl substituted piperazinyl.

In another aspect of the present invention, the present invention provides a compound represented by formula 1 below, a stereoisomer thereof, a solvate thereof, a hydrate thereof, or a pharmaceutically acceptable salt thereof. 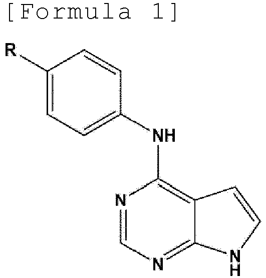 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl.

In another aspect of the present invention, the present invention provides a health functional food composition for preventing or ameliorating cancer comprising a compound represented by formula 1 or 2 above as an active ingredient.

In another aspect of the present invention, the present invention provides a method for treating cancer, comprising a step of administering a compound represented by formula 1 or 2 above to a subject in need thereof.

In another aspect of the present invention, the present invention provides a compound represented by formula 1 or 2 above for use in the prevention or treatment of cancer.

In another aspect of the present invention, the present invention provides a use of a compound represented by formula 1 or 2 above for the preparation of a medicament for use in the prevention or treatment of cancer.

### ADVANTAGEOUS EFFECT

The compound according to the present invention exhibits excellent anticancer effects against the triple-negative breast cancer cell line MDA-MB-231 as well as general cancer cell lines such as A549, SK-OV-3, SK-MEL-2, HCT15, etc., and thus can be advantageously used as an anticancer agent.

### BRIEF DESCRIPTION OF THE DRAWINGS

Figure 1 shows the MS spectrum of the compound prepared in Example 1.
Figure 2 shows the ¹H-NMR spectrum of the compound prepared in Example 1.
Figure 3 is a set of graphs showing the results of confirming the toxicity of the compound of Example 1 of the present invention to various cancer cell lines.
Figure 4a is a graph showing the change in body weight of mice according to administration of the compound of Example 1 to MDA-MB-231, a TNBC cell line.
Figure 4b is a graph showing the change in tumor volume in mice according to administration of the compound of Example 1 to MDA-MB-231, a TNBC cell line.
Figure 4c is a graph showing the change in tumor weight of mice according to administration of the compound of Example 1 to MDA-MB-231, a TNBC cell line.
Figure 5 is a graph showing the results of comparing the number of mitotic figure in tumor tissue according to administration of the compound of Example 1.
Figure 6 is a set of graphs showing the cell viability of BT20, a human-derived breast cancer cell line, after 24 hours of treatment with the compound of Example 2 in culture.
Figure 7 is a set of graphs showing the cell viability of BT20, a human-derived breast cancer cell line, after 48 hours of treatment with the compound of Example 2 in culture.
Figure 8 is a set of graphs showing the cell viability of MDA-MB-231, a TNBC cell line, after 24 hours of treatment with the compound of Example 2 in culture.
Figure 9 is a set of graphs showing the cell viability of MDA-MB-231, a TNBC cell line, after 48 hours of treatment with the compound of Example 2 in culture.
Figure 10 is a set of graphs showing the cell viability of MDA-MB-231, MDA-MB-453, and BT-20 cell lines after 24 hours of treatment with the compound of Example 3.
Figure 11 is a set of graphs showing the cell viability upon administration of the compound of Example 1 and cisplatin, alone and in combination, in tumor cells (SKOV-3 and OVCAR-3).
Figure 12 is a set of graphs showing the caspase-3 activity upon administration of the compound of Example 1 and sorafenib, alone and in combination, in tumor cells (SK Hep1 and Huh-7).

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In an aspect of the present invention, the present invention provides a pharmaceutical composition for preventing or treating cancer comprising a compound represented by formula 1 or 2 below, a stereoisomer thereof, a solvate thereof, a hydrate thereof, or a pharmaceutically acceptable salt thereof as an active ingredient. 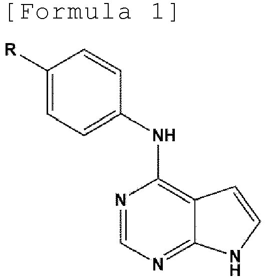 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl; 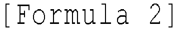 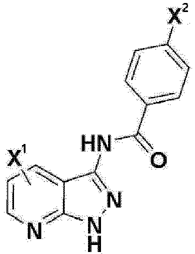
In formula 2 above,
X¹ is halogen, C₁₋₃ alkoxy, C₁₋₃ alkyl, cyano, hydroxy, amino, or piperidine substituted pyrazolyl,
X² is halogen, or methyl substituted piperazinyl.

In a preferred aspect, X¹ may be bromo or piperidine substituted pyrazolyl, and X² may be fluoro or methyl substituted piperazinyl.

In a further preferred aspect, a pharmaceutical composition for preventing or treating cancer comprising a compound represented by formula 1 or 2 above that is selected from the group consisting of the following compound group, a stereoisomer thereof, a solvate thereof, a hydrate thereof, or a pharmaceutically acceptable salt thereof is provided.
<1> N-(4-(pyrrolidin-1-yl)phenyl)-7H-pyrrolo[2,3-d]pyrimidin-4-amine;
<2> N-(5-bromo-1H-pyrazolo[3,4-b]pyridin-3-yl)-4-(4-methylpiperazin-1-yl)benzamide; and
<3> 4-fluoro-N-(5-(1-(piperidin-4-yl)-1H-pyrazol-4-yl)-1H-pyrazolo[3,4-b]pyridin-3-yl)benzamide.

In another aspect of the present invention, the pharmaceutical composition can be used in combination treatment with anticancer agents.

The anticancer agent can be at least one selected from the group consisting of cisplatin, sorafenib, opdivo, tecentriq, keytruda, imfinzi, OKN-007, gefitinib, doxorubicin, vinblastine, taxol, etoposide, 5-FU, and ifosfamide.

In another aspect of the present invention, the present invention provides a compound represented by formula 1 below, a stereoisomer thereof, a solvate thereof, a hydrate thereof, or a pharmaceutically acceptable salt thereof. 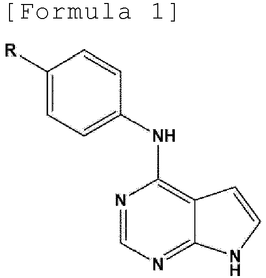 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl.

In a more preferred aspect,
R may be 5-6 membered heterocycloalkyl containing one or more heteroatoms selected from the group consisting of N, O and S, 5-6 membered heteroaryl containing one or more heteroatoms selected from the group consisting of N, O and S, or phenyl; or
R may be 5 membered heterocycloalkyl containing one or more heteroatoms selected from the group consisting of N, O and S, 5 membered heteroaryl containing one or more heteroatoms selected from the group consisting of N, O and S, or phenyl; or
R may be 5 membered heterocycloalkyl containing one N, or 5 membered heteroaryl containing at least one N; or
R may be 5 membered heterocycloalkyl containing one N.

Most preferably, the compound represented by formula 1 above may be a compound represented by formula 3 below.

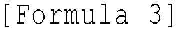

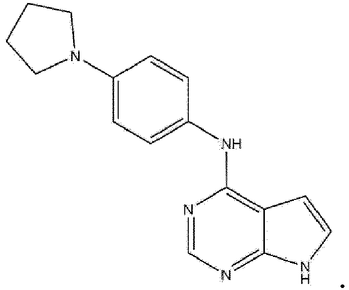

The compound represented by formula 3 above corresponds to the compound synthesized in <Example 1> described below.

In the present invention, the term "pharmaceutically acceptable salt" refers to salts commonly used in the pharmaceutical industry, for example, inorganic ionic salt made from calcium, potassium, sodium and magnesium; inorganic acid salts made from hydrochloric acid, nitric acid, phosphoric acid, bromic acid, iodic acid, perchloric acid, and sulfuric acid; organic acid salts made from acetic acid, trifluoroacetic, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc.; sulfonic acid salts made from methanesulfonic acid, ethanesulfonic acid, benzenesulfonic acid, p-toluenesulfonic acid, and naphthalenesulfonic acid; amino acid salts made from glycine, arginine, lysine, etc.; and amine salts made from trimethylamine, triethylamine, ammonia, pyridine, picoline, etc., but these salts do not limit the types of salts of the present invention.

In the present invention, the term "isomer" refers to a compound of the present invention or a salt thereof that has the same chemical formula or molecular formula but is structurally or sterically different. The isomer includes structural isomers such as tautomers and stereoisomers, and stereoisomers include both R or S isomers with asymmetric carbon centers (optical isomers, enantiomers) and geometric isomers (trans, cis). In the present invention, all stereoisomers of the compound represented by formula 1 or 2 and mixtures thereof are also included in the scope of the present invention.

In the present invention, the term "hydrate" refers to a compound represented by formula 1 above and water bound by non-covalent intermolecular forces, and may include a stoichiometric or non-stoichiometric amount of water. Specifically, the hydrate may contain water in a ratio of about 0.25 mole to about 10 mole based on 1 mole of the active ingredient, and more specifically may contain about 0.5 mole, about 1 mole, about 1.5 mole, about 2 mole, about 2.5 mole, about 3 mole, about 5 mole, etc.

In the present invention, the term "solvate" refers to a compound represented by formula 1 above and a solvent other than water bound by non-covalent intermolecular forces, and may include a stoichiometric or non-stoichiometric amount of water. Preferred solvents are volatile, non-toxic, and can be administered to humans in trace amounts. Specifically, the solvate may contain water in a ratio of about 0.25 mole to about 10 mole based on 1 mole of the active ingredient, and more specifically may contain about 0.5 mole, about 1 mole, about 1.5 mole, about 2 mole, about 3 mole, about 5 mole, etc.

In another aspect of the present invention, the present invention provides a pharmaceutical composition for preventing or treating cancer comprising a compound represented by formula 1 or 2 above, a stereoisomer thereof, a solvate thereof, a hydrate thereof, or a pharmaceutically acceptable salt thereof as an active ingredient.

In the present invention, the term "comprising (containing) as an active ingredient" means including in a dosage range that brings the effect of preventing, ameliorating, or treating cancer, and the dosage range may vary depending on the severity and formulation, and the number of applications may also vary depending on the age, weight, and physical constitution of the subject. In one embodiment of the present invention, in the pharmaceutical composition of the present invention, the compound represented by formula 1 is included, for example, in an amount of 0.001 mg/kg or more, preferably 0.1 mg/kg or more, more preferably 10 mg/kg or more, more preferably 100 mg/kg or more, more preferably 250 mg/kg or more, and most preferably 0.1 g/kg or more. The quantitative upper limit of the compounds represented by formula 1 included in the pharmaceutical composition of the present invention may be selected by those skilled in the art within an appropriate range.

The pharmaceutical composition according to the present invention may comprise an effective amount of the compound represented by formula 1 or 2 alone or may include one or more pharmaceutically acceptable carriers, excipients or diluents.

Examples of the carriers, excipients and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil, but not always limited thereto. In addition, fillers, anticoagulants, lubricants, wetting agents, fragrances, emulsifiers and preservatives may be additionally included.

The pharmaceutical composition of the present invention can be formulated using the methods known in the art to provide rapid, sustained or delayed release of the active ingredient after administration to a subject. The pharmaceutical composition can be formulated in the form of an oral preparation, an injectable preparation, or an external preparation. The oral preparation can be selected from the group consisting of tablets, pills, powders, granules, capsules, suspensions, solutions, emulsions, syrups and freeze-dried preparations, but not always limited thereto. In addition, the external preparation can be selected from the group consisting of creams, gels, ointments, emulsions, suspensions, sprays, and transdermal patches, but not always limited thereto.

The pharmaceutical composition of the present invention can be administered through various routes, including oral administration, transdermal administration, subcutaneous administration, intravenous administration, or intramuscular administration.

In another aspect of the present invention, the present invention provides a health functional food composition for preventing or ameliorating cancer comprising a compound represented by formula 1 or 2 above, a stereoisomer thereof, a solvate thereof, a hydrate thereof, or a pharmaceutically acceptable salt thereof as an active ingredient.

In the present invention, the term "amelioration" refers to alleviation, prevention, or treatment of cancer symptoms by administering, ingesting, or applying the pharmaceutical composition or food composition of the present invention to a subject suffering from cancer.

In the present invention, the term "prevention" refers to inhibiting or blocking cancer symptoms by administering, ingesting, or applying the pharmaceutical composition or food composition of the present invention to a subject not suffering from cancer.

In the present invention, the term "treatment" refers to complete cure of cancer symptoms as well as partial cure, improvement, and alleviation of cancer symptoms as a result of administering the pharmaceutical composition of the present invention to a subject suffering from cancer.

In the present invention, the term 'subject' refers to any animal, including humans, which has already developed or may develop cancer.

The pharmaceutical composition of the present invention is administered in a pharmaceutically effective amount.

In the present invention, the term "pharmaceutically effective amount" means an amount sufficient to treat a disease with a reasonable benefit/risk ratio applicable to medical treatment or improvement. The effective dose level depends on the factors including subject type and severity, age, gender, drug activity, sensitivity to drug, time of administration, administration route and excretion rate, duration of treatment, concomitant drugs, and other factors well known in the medical field.

In the present invention, the term "administration" means to provide a substance to a subject or patient by any appropriate method. The substance can be administered parenterally (e.g., intravenous injection, subcutaneous injection, intraperitoneal injection, or topical injection) or administered orally according to the desired method. The dosage range varies depending on the patient's weight, age, gender, health condition, diet, administration time, administration method, excretion rate, and severity of the disease. Specifically, in the present invention, the term "parenteral administration" refers to a method of administration subcutaneously, intramuscularly, intravenously, or intraperitoneally using a tube, excluding oral administration. In addition, in the present invention, the term "oral administration" refers to an administration method of injecting an agent for ameliorating pathological symptoms into the mouth.

For formulations for parenteral administration, sterilized suspensions, liquids, water-insoluble excipients, suspensions, emulsions, eye drops, ophthalmic ointments, syrups, suppositories, external preparations such as aerosols and sterilized injections can be prepared by the conventional method, and preferably pharmaceutical compositions of creams, gels, patches, sprays, ointments, plasters, lotions, liniments, ophthalmic ointments, eye drops, pastes or cataplasms can be prepared, but not always limited thereto. Water-insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

In the present invention, the cancer can be anyone selected from the group consisting of lung cancer, non-small cell lung cancer (NSCL), bronchial alveolar cell lung cancer, ovarian cancer, colorectal cancer, melanoma, stomach cancer, gastrointestinal cancer, liver cancer, bone cancer, pancreatic cancer, skin cancer, head and neck cancer, skin or eye melanoma, uterine cancer, rectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, multiple myeloma, and chronic or acute leukemia, and preferably the cancer can be liver cancer, ovarian cancer, lung cancer, colorectal cancer, melanoma, or breast cancer.

The food composition according to the present invention can be formulated in the same way as the pharmaceutical composition and used as a functional food or added to various foods. Food to which the food composition of the present invention can be added is exemplified by beverages, alcoholic beverages, confectionery, diet bars, dairy products, meat, chocolate, pizza, ramen, other noodles, gum, ice cream, and hangover relievers (drinks, low-viscosity gel type, pills, tablets, capsules, etc.), vitamin complexes, health supplements, etc.

The food composition of the present invention may include a compound represented by formula 1 or 2, a pharmaceutically acceptable salt thereof, a hydrate thereof, or a solvate thereof as an active ingredient, as well as ingredients commonly added during food production, for example, proteins, carbohydrates, fats, nutrients, seasonings and flavoring agents. Examples of the above-mentioned carbohydrates include monosaccharides such as glucose, fructose, etc.; disaccharides such as maltose, sucrose, oligosaccharides, etc.; and polysaccharides such as common sugars including dextrin and cyclodextrin, and sugar alcohols such as xylitol, sorbitol, and erythritol. Natural sweetening agents (thaumatin, stevia extract, for example rebaudioside A, glycyrrhizin, etc.) and synthetic sweetening agents (saccharin, aspartame, etc.) can be included as a sweetening agent. When the food composition of the invention is formulated as drinks and beverages, it may additionally include citric acid, high fructose corn syrup, sugar, glucose, acetic acid, malic acid, fruit juice, and various plant extracts in addition to a compound represented by formula 1 or 2, a pharmaceutically acceptable salt thereof, a hydrate thereof or a solvate thereof.

The present invention provides a health functional food or health supplement food containing the food composition comprising a compound represented by formula 1 or 2, a pharmaceutically acceptable salt thereof, a hydrate thereof, or a solvate thereof as an active ingredient. In the present invention, the term "health functional food or health supplement food" refers to a food manufactured and processed using raw materials or ingredients that have functional properties useful to the human body in accordance with the Act on Health Functional Food. The term "functional" food refers to a food consumed for the purpose of obtaining useful health effects such as regulating nutrients for the structure and function of the human body or physiological effects. In the present invention, the functional food is a food product prepared by adding a compound represented by formula 1, a pharmaceutically acceptable salt thereof, a hydrate thereof, or a solvate thereof to food materials such as beverages, teas, spices, gum, and confectionery, or by encapsulating, powdering, or suspending. The above functional food has specific health effects when consumed, but unlike general drugs, it has the advantage of not having any side effects that may occur when taking the drug for a long time because it is manufactured using food as a raw material. The health functional food or health supplement food of the present invention obtained in this way is very useful because it can be taken on a daily basis. The amount of a compound represented by formula 1, a pharmaceutically acceptable salt thereof, a hydrate thereof, or a solvate thereof in such a health functional food or health supplement food cannot be uniformly prescribed because it depends on the type of the health functional food being targeted, but it can be added in a range that does not impair the original taste of the food, and is generally in the range of 0.01 to 50 wt%, preferably 0.1 to 20 wt% of the target food. In addition, in the case of the health functional food or health supplement food in the form of pills, granules, tablets, or capsules, it is usually added in the range of 0.1 to 100 wt%, preferably 0.5 to 80 wt%. In one embodiment of the present invention, the health functional food or health supplement food of the present invention may be in the form of a pill, tablet, capsule, or beverage.

The food composition of the present invention can contain a conventional food additive. The suitability as a 'food additive' is determined by the standards and criteria for the item in question in accordance with the general rules and general test methods for food additives approved by the Ministry of Food and Drug Safety, unless otherwise specified.

The items listed in the "Korean Food Additives Code" include, for example, chemical compounds such as ketones, glycine, potassium citrate, nicotinic acid, and cinnamic acid; natural additives such as persimmon color, licorice extract, crystalline cellulose, sorghum color, and guar gum; and mixed preparations such as sodium L-glutamate preparations, alkali agents for noodles, preservative formulations, and tar color formulations.

In addition, the food composition of the present invention can be prepared and processed in the form of tablets, capsules, powders, granules, liquids, pills, and the like, for the purpose of preventing and/or ameliorating cancer.

For example, the health functional food in tablet form can be prepared by granulating a mixture of a food composition comprising a compound represented by formula 1 or 2, a pharmaceutically acceptable salt thereof, a hydrate thereof, or a solvate thereof as an active ingredient, an excipient, a binder, a disintegrant, and other additives in a conventional manner, and then compression molding the mixture with a lubricant or the like, or directly compression molding the mixture. In addition, the health functional food in tablet form can contain a flavors enhancer or the like as needed, and can also be coated with a suitable coating agent as needed.

Among the health functional food in capsule form, hard capsules can be prepared by filling conventional hard capsules with a mixture of a food composition comprising a compound represented by formula 1 or 2, a pharmaceutically acceptable salt thereof, a hydrate thereof, or a solvate thereof as an active ingredient, and additives such as excipients, or a granularity thereof or a coated granularity thereof. Soft capsules can be prepared by filling a mixture of a food composition comprising a compound represented by formula 1, a pharmaceutically acceptable salt thereof, a hydrate thereof, or a solvate thereof as an active ingredient, and additives such as excipients into a capsule base such as gelatin. The soft capsules can contain plasticizers such as glycerin or sorbitol, colorants, preservatives, etc., if necessary.

The health functional food in pill form can be prepared by molding a mixture of a food composition comprising a compound represented by formula 1 or 2, a pharmaceutically acceptable salt thereof, a hydrate thereof, or a solvate thereof as an active ingredient, an excipient, a binder, a disintegrant, and the like in any suitable manner, and if necessary, it can be coated with white sugar or another suitable coating agent, or coated with starch, talc, or other suitable substances.

The health functional food in granule form can be prepared by using a mixture of a food composition comprising a compound represented by formula 1 or 2, a pharmaceutically acceptable salt thereof, a hydrate thereof, or a solvate thereof as an active ingredient, an excipient, a binder, a disintegrant, and the like in any suitable manner, and if necessary, it can contain flavoring agents, flavors enhancers, etc. When a particle size test was conducted using No. 12 (1680 µm), No. 14 (1410 µm), and No. 45 (350 µm) sieves, the entire amount of health functional food in granule form passed through the No. 12 sieve, less than 5.0% of the total amount remained in the No. 14 sieve, and less than 15.0% of the total amount passed through the No. 45 sieve.

There is no particular limitation on the type of food, and it includes all health functional foods in the conventional sense.

The matters mentioned in the pharmaceutical composition and food composition of the present invention are applied in the same manner unless they are mutually contradictory.

In another aspect of the present invention, the present invention provides a method for preventing or treating cancer, comprising a step of administering a compound represented by formula 1 or 2 described herein, a stereoisomer thereof, a solvate thereof, a hydrate thereof, or a pharmaceutically acceptable salt thereof to a subject in need thereof.

In another aspect of the present invention, the present invention provides a method for preventing or treating cancer, comprising a step of co-administering a compound represented by formula 1 or 2 described herein, a stereoisomer thereof, a solvate thereof, a hydrate thereof, or a pharmaceutically acceptable salt thereof and radiation or anticancer agent to a subject in need thereof.

In another aspect of the present invention, the present invention provides a use of a compound represented by formula 1 or 2 described herein, a stereoisomer thereof, a solvate thereof, a hydrate thereof, or a pharmaceutically acceptable salt thereof for the preparation of a medicament for use in the prevention or treatment of cancer.

In another aspect of the present invention, the present invention provides a use of a compound represented by formula 1 or 2 described herein, a stereoisomer thereof, a solvate thereof, a hydrate thereof, or a pharmaceutically acceptable salt thereof and radiation or anticancer agent, which are administered in combination, for the preparation of a medicament for use in the prevention or treatment of cancer.

For the above method or use, the detailed description of the pharmaceutical composition described above may be applied.

The compound according to the present invention exhibits excellent anticancer effects against the triple-negative breast cancer cell line MDA-MB-231 as well as general cancer cell lines such as A549, SK-OV-3, SK-MEL-2, HCT15, etc., and thus can be advantageously used as an anticancer agent, which is supported by the following examples and experimental examples.

Hereinafter, the present invention will be described in detail by the following examples and experimental examples.

However, the following examples and experimental examples are only for illustrating the present invention, and the contents of the present invention are not limited thereto.

### Sample preparation

### List of Reagents with Supplier and Lot Number

| **Material** | **Supplier/Cat No** | **Let No** |
|---|---|---|
| 4-Chloro-7H-pyrrolo[2,3-d]pyrimidine (1) | Combi Block, OS-7803 | B38030 |
| 4-Pyrrolidin-1-ylamline (2) | BLD Pharm, BD100212 | BKXS36 |
| 1-Butanol (n-BuOH) | Samchun, 81125 | 121218 |
| Trifluoroecetic acid (TFA) | Aldrich, 302031 | STBH6629 |
| Acetonitrile | Samchun, A1764 | 060819 |
| Dimethylsulfoxide (DMSO) | Samchun, D0458 | 050919 |
| *N*,*N*-Diisopropylethylamine (DIPEA) | Tokyo Chemical Industry, D1599 | HGU7J-AQ |
| Dimethylsulfoxide-d₆ (DMSO-d₆) | Sunfine Global | 1002814 |
| Ethyl acetate (EtO Ac) | Samchun, E0191 | 061919 |
| Methanol | Samchun, M0584 | 052019 |
| Water | Samchun, W0054 | 051419 |

### Example 1: N-(4-(pyrrolidin-1-yl)phenyl)-7H-pyrrolo[2,3-d]pyrimidin-4-amine

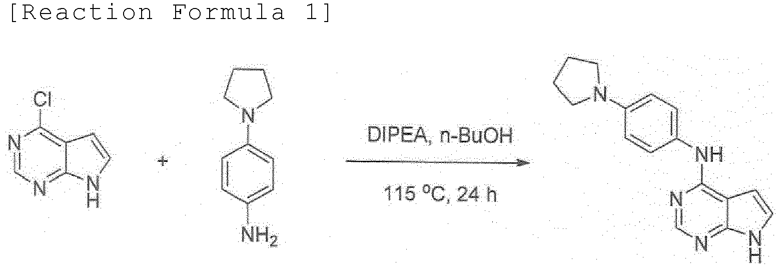

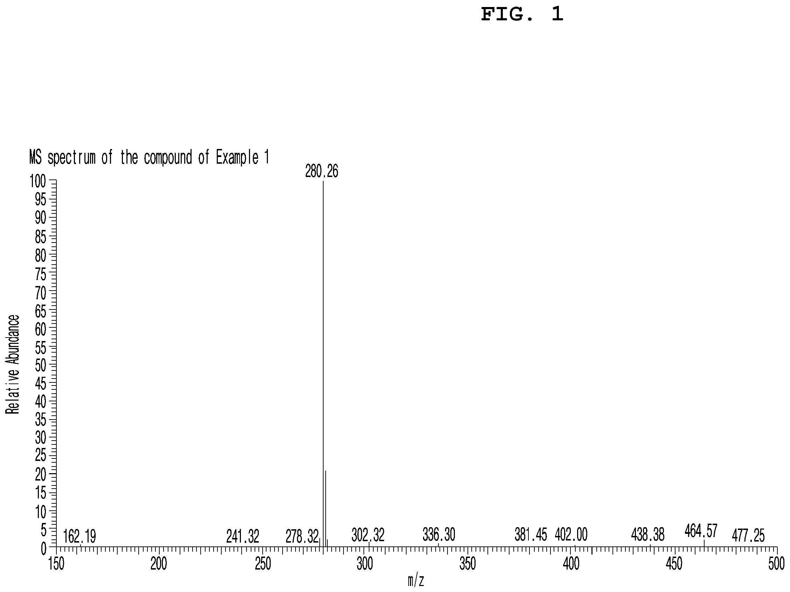

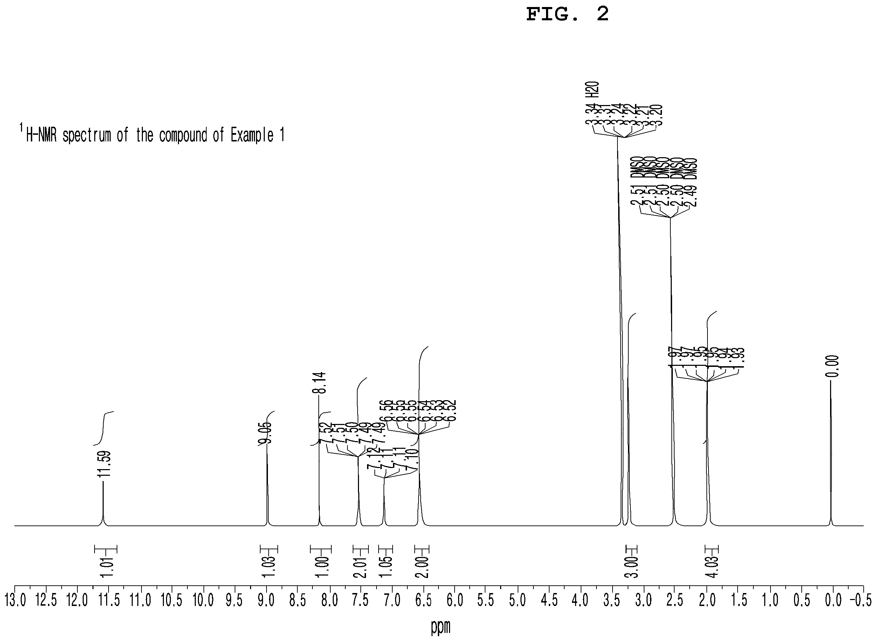

DIPEA (9.78 mL, 56.0 mmol, N,N-diisopropylethylamine) was added to a solution of 4-chloro-7H-pyrrolo[2,3-d]pyrimidine (4.30 g, 28.0 mmol) and 4-pyrrolidin-1-ylaniline (5.00 g, 30.8 mmol) dissolved in 1-butanol (86 mL). Under a nitrogen atmosphere, the mixed solution was stirred at 115°C for 24 hours. The suspension was cooled to room temperature, stirred for 1 hour, filtered, and washed with 1-butanol (10 mL). The obtained solid was treated with EtOAc (100 mL) and water (100 mL) and stirred for 18 hours. The suspension was filtered, washed with water (10 mL) and EtOAc (10 mL), and dried under reduced pressure to give the title compound (4.41 g, 56%) as a brown solid. The MS spectrum of the compound prepared in Example 1 is shown in Figure 1 and the 1H-NMR spectrum is shown in Figure 2. As shown in Figures 1 and 2, it was confirmed that the compound of Example 1 was well synthesized.

NMR spectra were recorded with Bruker BioSpin 400 MHz (AVANCE III HD 400) at room temperature, and upon sampling, each sample was dissolved in DMSO-d₆ and transferred to an NMR tube (5-mm o.d., Wilmad, WG-1007-7).

LC-MS was performed in Ion-trap Mass Spectrometer on Accela-Thermo Finnigan LCQ Fleet operating APCI(+) ionization mode.

Agilent 1100 series was used for HPLC, and the conditions were as follows.

### HPLC (Agilent 1100 series)

| | | | | | | |
|---|---|---|---|---|---|---|
| Column | : YMC Triart C18 (150 × 4.6 mm, 5 µm) | | | | | |
| Column Temperature | : 30 °C | | | | | |
| Mobile Phase | : A = 0.1 % TFA Water (v/v) | | | | | |
| | B = 0.1 % TFA Acetonitrile (v/v) | | | | | |
| Gradient Condition | : Time (min) | 0 | 20 | 25 | 25.1 | 30 |
| | B conc. (%) | 10 | 90 | 90 | 10 | 10 |
| Flow Rate | : 1.0 mL/min | | | | | |
| Concentration of Sample | : 0.5 mg/mL | | | | | |
| Dissolving Solvent | : DMSO/Methanol = 20/80 (v/v) | | | | | |
| Injection Volume | : 10 µL | | | | | |
| Detector | : UV 300 nm | | | | | |
| Chemical purity is 99.5 %, RT = 9.37 min | | | | | | |

### Example 2: N-(5-bromo-1H-pyrazolo[3,4-b]pyridin-3-yl)-4-(4-methylpiperazin-1-yl)benzamide

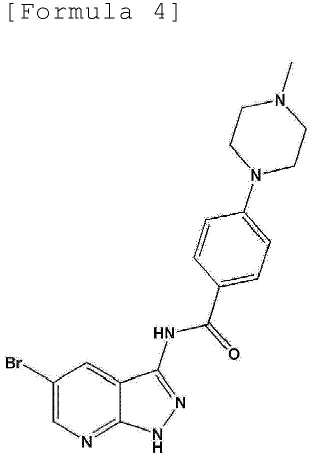

The compound of Example 2 was prepared with reference to Korean Patent No. 10-2129114.

### Example 3: 4-fluoro-N-(5-(1-(piperidin-4-yl)-1H-pyrazol-4-yl)-1H-pyrazolo[3,4-b]pyridin-3-yl)benzamide

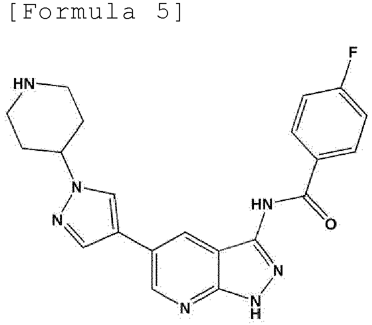

The compound of Example 3 was prepared with reference to Korean Patent No. 10-2129114.

### Example 3: Evaluation of cytotoxicity on various cancer cell lines

The cytotoxicity of the compound of Example 1 against various cancer cell lines was evaluated. Etoposide, sold as an anticancer agent, was used as a control group drug.

Specifically, the cell viability assay was performed as follows.

A549, SK-OV-3, SK-MEL-2, and HCT15 cell lines were seeded in 96 well plates at the density of 5×10³ cells per well and cultured for 24 hours. Then, the plates were treated with the compound of Example 1 or etoposide at different concentrations, followed by culture for 24 and 48 hours. At the end of the culture, 100 *µ*ℓ of WST-8 solution was added to each well of the plate, and the absorbance was measured with a microplate reader using a 450 nm optical filter to measure the formazan produced by the reducing factors in living cells.
- A549: human non-small cell lung cancer cells
- SK-OV-3: human ovarian cancer cells
- SK-MEL-2: human melanoma cells
- HCT15: human colorectal cancer adenocarcinoma cells

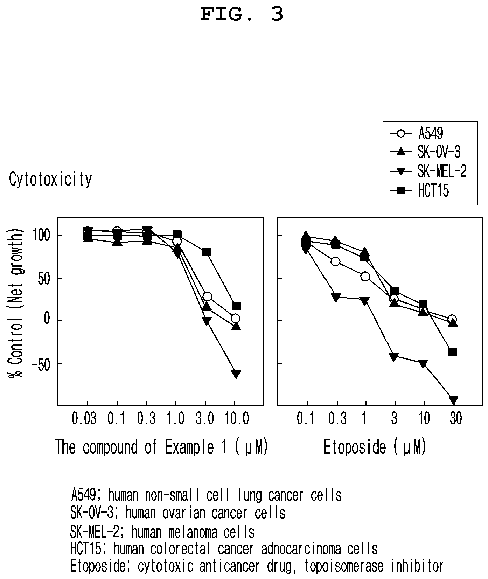

The results are shown in Figure 3.

Figure 3 is a set of graphs showing the results of confirming the toxicity of the compound of Example 1 of the present invention to various cancer cell lines.

As shown in Figure 3, the compound of Example 1 according to the present invention showed excellent toxicity to the cancer cell lines A549, SK-OV-3, SK-MEL-2, and HCT15 dose dependently, and thus it can be effectively used as an active ingredient in anticancer agents.

From the above results, it was confirmed that the compound of Example 1 according to the present invention exhibits excellent toxicity to various cancer cell lines, and is equally or more effective than etoposide, a topoisomerase inhibitor widely used in lung cancer, ovarian cancer, colorectal cancer, melanoma, etc., so the compound of the present invention can be effectively used as an active ingredient in various anticancer agents.

### Experimental Example 2: Evaluation of breast cancer growth inhibition

An experiment was performed to confirm the tumor inhibition of the compound of Example 1, the active ingredient of the pharmaceutical composition of the present invention, on a xenograft tumor sample of breast cancer cells (MBA-MB-231), a human-derived TNBC cell line.

Specifically, to confirm the effect of the compound of Example 1, which is the active ingredient of the pharmaceutical composition provided by the present invention, on the inhibition of breast cancer cell growth, an experiment was performed using 80 female 5-week-old xenograft Balb/c nude mice. The experimental xenograft Balb/c nude mice lacks T cells, the immune cells, were selected as an animal model suitable for transplanting human cancer cells. At the time of administration, animals within ±20% of the total average body weight were selected, and tumor growth was checked twice a week on the surface, and the size of the tumor was measured with a vernier caliper for 3 weeks. In addition, the compound of Example 1 was administered intravenously at a concentration of 25 mpk, and 30% PEG was used as a solvent.

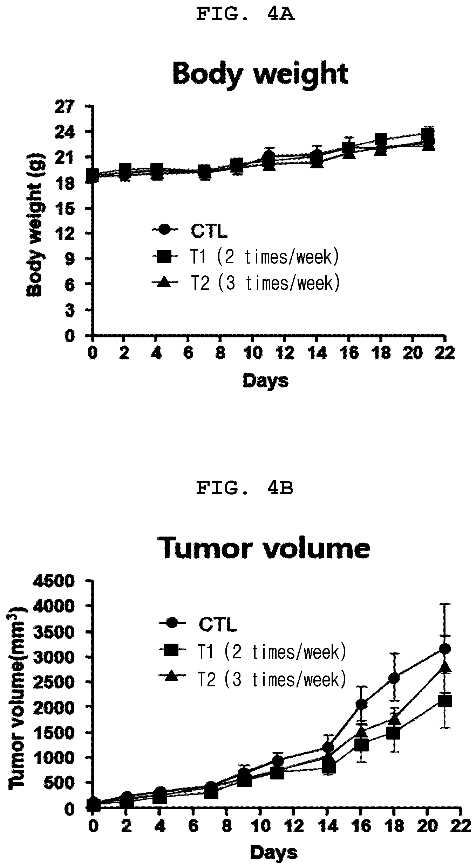

The results are shown in Figures 4a to 4c.

Figures 4a to 4c are graphs showing the results of evaluating the growth inhibitory activity of the compound of Example 1 on MDA-MB-231, a TNBC cell line.

From Figure 4a, which shows the change in body weight according to the administration of the compound of Example 1, it was confirmed that the compound of Example 1 had no biological toxicity and was safe. In particular, the cachexia phenomenon, which is commonly observed following the administration of anticancer agents, was not observed.

From Figure 4b, which shows the change in tumor size according to the administration of the compound of Example 1, it was confirmed that tumor growth was significantly suppressed in the group administered with the compound of Example 1 compared to the group not administered with the compound of Example 1 (CTL).

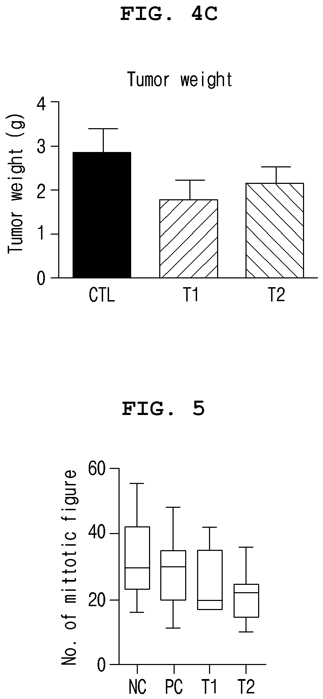

From Figure 4c, which shows the change in tumor weight according to the administration of the compound of Example 1, it was confirmed that tumor growth was significantly suppressed in the group administered with the compound of Example 1 compared to the group not administered with the compound of Example 1 (CTL).

From the above results, it was found that the compound of Example 1 according to the present invention is highly active in suppressing the growth of triple-negative breast cancer, as well as various general cancer cell lines, as previously demonstrated in <Experimental Example 1>.

### Experimental Example 3: Comparison of number of mitotic figures

An experiment was performed to evaluate the tumor necrosis effect of the compound of Example 1 by measuring the number of apoptotic bodies in tumors and H&E staining of tumor tissues in BALB/c-nude mice injected with cancer cell lines. Specifically, to measure the number of apoptotic bodies in the tumor tissue of the T1 (2 times/week) and T2 (3 times/week) groups of <Experimental Example 2>, as shown in Figures 4a to 4c, five areas where the tumor tissues were growing were randomly selected and the number of apoptotic bodies was measured at x400 magnification. After calculating the sum of the measurements and expressing them as individual values, statistical comparative analysis was performed using GraphPad Prism 5.

As a result of observing the apoptotic body, it was observed that the cells were shrunk and divided (blebs) and that empty space (hollow) surrounded the periphery. In addition, the eosinophilicity of the cytoplasm was increased, and the nucleus was small, concentrated or fragmented, and was observed to be phagocytosed by surrounding cells. Since the frequency of mitotic figures in tumor tissue is related to the dividing ability of tumor cells, it can reflect the degree of malignancy of the tumor.

The results are shown in Table 1 and Figure 5 below.

Figure 5 is a graph showing the results of comparing the number of mitotic figures in tumor tissue according to administration of the compound of Example 1.

**[Table 1]**

| | NC | PC | T1 | T2 |
|---|---|---|---|---|
| No. examined | 10 | 10 | 10 | 10 |
| Mitotic index* | 31.9 ± 12.1 | 28.3 ± 12.1 | 25.5 ± 10.2 | 20.8± 7.4 |

According to the number of apoptotic bodies confirmed in Table 1 and Figure 5, the number was higher in the T1 and T2 groups treated with the compound of Example 1, the active ingredient of the pharmaceutical composition of the present invention, compared to NC (Negative Control) dose dependently, confirming that the compound of Example 1 of the present invention had a tumor suppressive effect by increasing apoptosis of cancer cells.

### Experimental Example 4: Evaluation of breast cancer cell line inhibition of Example 2 and Example 3 compounds

An experiment was performed to confirm the growth inhibitory effect of the compounds of Examples 1 and 2, the active ingredients of the pharmaceutical composition of the present invention, on breast cancer cell lines through cell viability assay.

Specifically, the cell viability assay was performed as follows. BT20, a human-derived breast cancer cell line, and MDA-MB-231, a TNBC cell line, were each seeded in 96-well plates at the density of 5×10³ cells/well and cultured for 24 hours. Then, I-bet 762 and the compounds were treated thereto at different concentrations and cultured for 48 hours. At the end of the culture, 100 *µ*ℓ of WST-8 solution was added to each well of the plate, and the absorbance was measured with a microplate reader using a 450 nm optical filter to measure the formazan produced by the reducing factors in living cells.

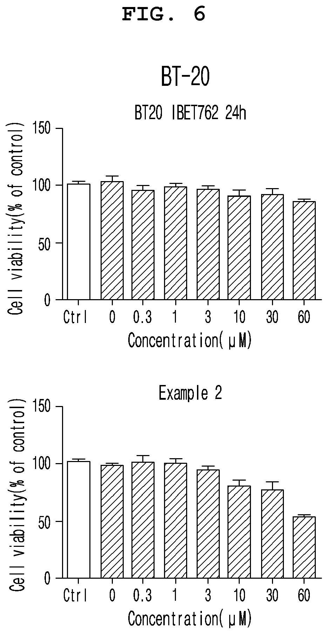

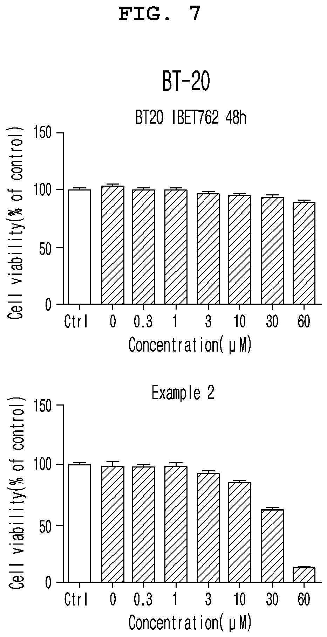

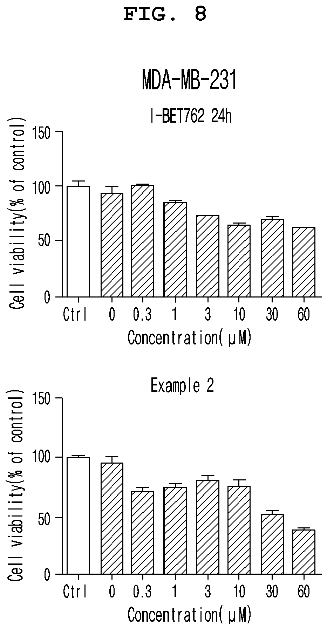

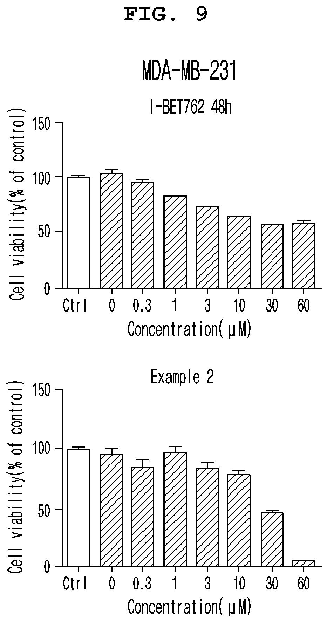

As shown in Figures 6 to 9, as a result of analyzing the formation of mammospheres by culturing the human-derived breast cancer cell line BT20 and the TNBC cell line MDA-MB-231, it was confirmed that the compound of Example 2, the active ingredient of the pharmaceutical composition of the present invention, inhibited the cell viability not only in a general breast cancer cell line, but also in a TNBC cell line dose dependently.

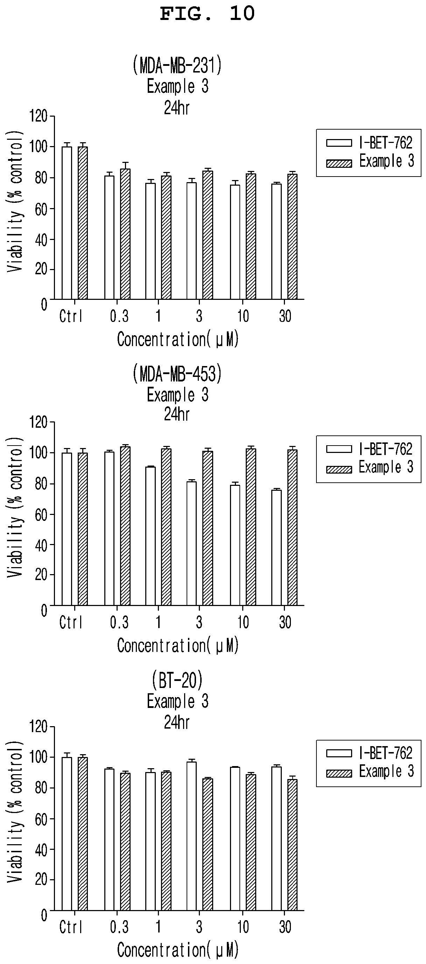

Figure 10 shows the cell viability of MDA-MB-231, MDA-MB-453, and BT-20 after 24 hours of treatment with the compound of Example 3. As shown in Figure 10, it was confirmed that the cell viability was suppressed not only in general breast cancer cell lines, but also in TNBC cell lines dose dependently.

### Experimental Example 5: Evaluation of efficacy in combination with other anticancer agents

### <5-1> Evaluation of efficacy in combination with cisplatin

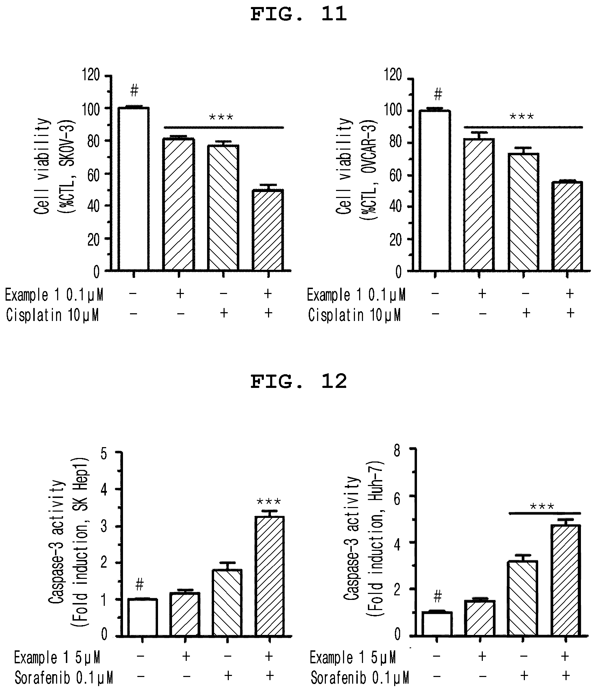

Human ovarian cancer cell lines (SKOV-3 and OVCAR-3) were treated with control (no treatment), 0.1 µM of the compound of Example 1, 10 µM of cisplatin, and 0.1 µM of the compound of Example 1 / 10 µM of cisplatin, and the cell viability of SKOV-3 and OVCAR-3 was confirmed. The results are shown in Figure 11.

It was confirmed that the cell viability was suppressed when 0.1 µM of the compound of Example 1 or 1 µM of cisplatin was administered to SKOV-3 and OVCAR-3 cell lines. In addition, it was also confirmed that when 0.1 µM of the compound of Example 1 and 10 µM of cisplatin were administered in combination, the cell viability was further inhibited (synergistic effect).

### <5-2> Evaluation of efficacy in combination with sorafenib

Human liver cancer cell lines (SK Hep1 and Huh-7) were treated with control (no treatment), 5 µM of the compound of Example 1, 0.1 µM of sorafenib, and 5 µM of the compound of Example 1 / 0.1 µM of sorafenib, and the cell viability of SK Hep1 and Huh-7 was confirmed. The results are shown in Figure 12.

It was confirmed that the cell viability was suppressed when 5 µM of the compound of Example 1 or 0.1 µM of sorafenib was administered to SK Hep1 and Huh-7 cell lines. In addition, it was also confirmed that when 5 µM of the compound of Example 1 and 0.1 µM of sorafenib were administered in combination, that caspase-3 activity was further inhibited (synergistic effect).

## Claims

1. A pharmaceutical composition for use in preventing or treating cancer comprising a compound represented by formula 1 or 2 below, a stereoisomer thereof, a solvate thereof, a hydrate thereof, or a pharmaceutically acceptable salt thereof as an active ingredient: 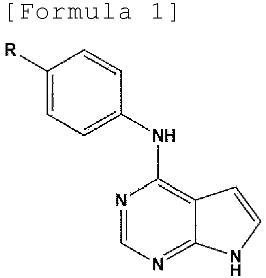 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl; 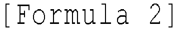 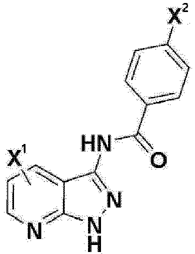
In formula 2 above,
X¹ is halogen, C₁₋₃ alkoxy, C₁₋₃ alkyl, cyano, hydroxy, amino, or piperidine substituted pyrazolyl, and
X² is halogen, or methyl substituted piperazinyl.

2. The pharmaceutical composition for use in preventing or treating cancer according to claim 1, wherein the X¹ is bromo, or piperidine substituted pyrazolyl, and the X² is fluoro, or methyl substituted piperazinyl.

3. The pharmaceutical composition for use in preventing or treating cancer according to claim 1, wherein the pharmaceutical composition comprises a compound represented by formula 1 or 2 selected from the group consisting of the following compound group, a stereoisomer thereof, a solvate thereof, a hydrate thereof, or a pharmaceutically acceptable salt thereof:
<1> N-(4-(pyrrolidin-1-yl)phenyl)-7H-pyrrolo[2,3-d]pyrimidin-4-amine;
<2> N-(5-bromo-1H-pyrazolo[3,4-b]pyridin-3-yl)-4-(4-methylpiperazin-1-yl)benzamide; and
<3> 4-fluoro-N-(5-(1-(piperidin-4-yl)-1H-pyrazol-4-yl)-1H-pyrazolo[3,4-b]pyridin-3-yl)benzamide.

4. The pharmaceutical composition for use in preventing or treating cancer according to claim 1, wherein the cancer is at least one selected from the group consisting of lung cancer, non-small cell lung cancer (NSCL), bronchial alveolar cell lung cancer, ovarian cancer, colorectal cancer, melanoma, stomach cancer, gastrointestinal cancer, liver cancer, bone cancer, pancreatic cancer, skin cancer, head and neck cancer, skin or eye melanoma, uterine cancer, rectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, multiple myeloma, and chronic or acute leukemia.

5. The pharmaceutical composition for use in preventing or treating cancer according to claim 4, wherein the breast cancer is triple-negative breast cancer.

6. The pharmaceutical composition for use in preventing or treating cancer according to claim 1, wherein the pharmaceutical composition is for use in combination therapy with anticancer agents.

7. The pharmaceutical composition for use in preventing or treating cancer according to claim 6, wherein the anticancer agent is at least one selected from the group consisting of cisplatin, sorafenib, opdivo, tecentriq, keytruda, imfinzi, OKN-007, gefitinib, doxorubicin, vinblastine, taxol, etoposide, 5-FU, and ifosfamide.

8. A health functional food composition for use in preventing or ameliorating cancer comprising a compound represented by formula 1 or 2 below, a stereoisomer thereof, a solvate thereof, a hydrate thereof, or a pharmaceutically acceptable salt thereof as an active ingredient: 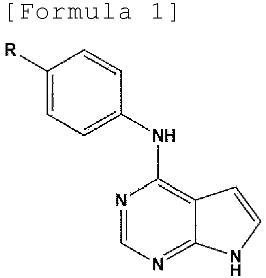 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl; 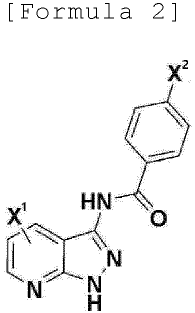
In formula 2 above,
X¹ is halogen, C₁₋₃ alkoxy, C₁₋₃ alkyl, cyano, hydroxy, amino, or piperidine substituted pyrazolyl,
X² is halogen, or methyl substituted piperazinyl.

9. A compound represented by formula 1 below, a stereoisomer thereof, a solvate thereof, a hydrate thereof, or a pharmaceutically acceptable salt thereof: 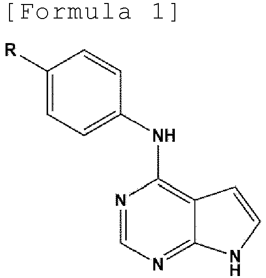 In formula 1 above,
R is 5-10 membered heterocycloalkyl containing one or more heteroatoms selected from the group consisting of N, O and S, 5-10 membered heteroaryl containing one or more heteroatoms selected from the group consisting of N, O and S, or C₆₋₁₀ aryl.

10. A method for preventing or treating cancer, comprising a step of administering a compound represented by formula 1 or 2 of claim 1, a stereoisomer thereof, a solvate thereof, a hydrate thereof, or a pharmaceutically acceptable salt thereof to a subject in need thereof.

11. A method for preventing or treating cancer, comprising a step of co-administering a compound represented by formula 1 or 2 of claim 1, a stereoisomer thereof, a solvate thereof, a hydrate thereof, or a pharmaceutically acceptable salt thereof and radiation or anticancer agent to a subject in need thereof.

12. A use of a compound represented by formula 1 or 2 of claim 1, a stereoisomer thereof, a solvate thereof, a hydrate thereof, or a pharmaceutically acceptable salt thereof for the preparation of a medicament for use in the prevention or treatment of cancer.

13. A use of a compound represented by formula 1 or 2 of claim 1, a stereoisomer thereof, a solvate thereof, a hydrate thereof, or a pharmaceutically acceptable salt thereof and anticancer agents, which are administered in combination, for the preparation of a medicament for use in the prevention or treatment of cancer.
